# EUROPEAN PATENT APPLICATION

(11) **EP 3 591 023 A1**
(43) Date of publication of application: **08.01.2020**
(21) Application number: 18761371.6
(22) Date of filing: 02.03.2018
(51) Int. Cl.: C09K 5/16, F28D 20/00

(54) **HEAT STORAGE MATERIAL COMPRISING METAL SALT OF CYANURIC ACID**

(30) Priority: 03.03.2017 JP 2017040802
(71) Applicant: Nissan Chemical Corporation, Tokyo 103-6119 (JP)
(72) Inventor: YOSHINO, Hironobu, Funabashi-shi Chiba 274-0052 (JP)
(74) Representative: Hoffmann Eitle
(86) International application number: PCT/JP2018/008092
(87) International publication number: WO 2018/159828

(57) **Abstract**

There is provided a heat-storage material, in particular a chemical heat-storage material that adsorbs or desorbs water vapor (water) at a low temperature (i.e., usable at a low temperature) and stores a large amount of heat. A chemical heat-storage material comprising a cyanuric acid metal salt, wherein the chemical heat-storage material generates or absorbs heat by adsorption or desorption of water vapor (water).

## Description

### TECHNICAL FIELD

The present invention relates to a heat-storage material, in particular a chemical heat-storage material. More particularly, the present invention relates to a chemical heat-storage material that adsorbs or desorbs water vapor (water) at a low temperature and stores a large amount of heat.

### BACKGROUND ART

The term "heat-storage material" collectively refers to materials that store heat energy. Some heat-storage materials are known to absorb or release heat. Such known heat-storage materials include a sensible heat-storage material that stores heat energy by the heat capacity of the material through application of a temperature difference; a latent heat-storage material that stores heat by the transfer of heat energy during the phase change of the material; and a chemical heat-storage material that stores heat of chemical reaction generated upon contact between a reaction medium and the heat-storage material. Among these heat-storage materials, a chemical heat-storage material is advantageous in that it has a large heat storage capacity, can generate heat at a constant temperature, and can be stored at ambient temperature if a reactant is separated therefrom.

Calcium oxide is a typical example of the aforementioned chemical heat-storage material and is known to release or absorb heat in association with hydration or dehydration reaction.

Another chemical heat-storage material which contains a coordination polymer composed of a metal element and an organic ligand has been proposed (Patent Document 1). Patent Document 1 discloses that a compound obtained by reaction of a mixture of copper (II) nitrate trihydrate, isonicotinic acid, and water exhibits a desorption temperature of water of 63.5°C and a heat absorption amount (heat storage amount) of 309 J/g as determined with a differential scanning calorimeter.

### Prior Art Documents

### Patent Documents

Patent Document 1: Japanese Patent Application Publication No. 2005-097530 (JP 2005-097530 A)

### SUMMARY OF THE INVENTION

### Problems to be Solved by the Invention

When the aforementioned calcium oxide is used as a chemical heat-storage material, the material generates heat by hydration reaction even at a low temperature of 30°C or lower and effectively releases heat. However, the dehydration reaction of calcium hydroxide generated by the hydration reaction (i.e., heat storage) requires a high temperature of 400°C or higher. Thus, calcium oxide poses a problem in terms of practical use.

When the coordination polymer composed of a metal element and an organic ligand disclosed in Patent Document 1 is used as a chemical heat-storage material, the material can be utilized at a temperature of 200°C or lower, but the material exhibits a heat storage amount smaller than that of the aforementioned calcium oxide system. Thus, the coordination polymer poses a problem in that it has an insufficient performance as a heat-storage material.

An object of the present invention is to provide a chemical heat-storage material for solving the aforementioned problems; specifically, a chemical heat-storage material that adsorbs or desorbs water vapor (water) at a low temperature (i.e., usable at a low temperature) and stores a large amount of heat.

### Means for Solving the Problems

The present inventor has conducted extensive studies for solving the aforementioned problems, and as a result has found that a cyanuric acid metal salt can be used as a chemical heat-storage material that adsorbs or desorbs water vapor (water) at a low temperature, stores a large amount of heat, and thus exhibits a good performance. The present invention has been accomplished on the basis of this finding.

Accordingly, a first aspect of the present invention is a chemical heat-storage material comprising a cyanuric acid metal salt, wherein the chemical heat-storage material generates or absorbs heat by adsorption or desorption of water vapor (water).

A second aspect of the present invention is the chemical heat-storage material according to the first aspect, wherein the metal species of the metal salt is at least one selected from the group consisting of lithium, sodium, potassium, magnesium, calcium, strontium, barium, aluminum, manganese, iron, cobalt, copper, nickel, zinc, silver, and tin.

A third aspect of the present invention is the chemical heat-storage material according to the second aspect, wherein the metal species of the metal salt is an alkaline earth metal.

A fourth aspect of the present invention is the chemical heat-storage material according to the second aspect, wherein the metal species of the metal salt is at least one selected from the group consisting of magnesium and calcium.

A fifth aspect of the present invention is the chemical heat-storage material according to the fourth aspect, wherein the ratio by mole of cyanuric acid to at least one selected from the group consisting of magnesium and calcium is 2 : 1.

A sixth aspect of the present invention is the chemical heat-storage material according to any one of the first to fifth aspects, wherein the amount of heat generated by adsorption of water vapor (water) or the amount of heat absorbed by desorption of water vapor (water) is 0.5 MJ/kg or more.

A seventh aspect of the present invention is the chemical heat-storage material according to any one of the first to sixth aspects, wherein the chemical heat-storage material exhibits a desorption temperature of water vapor (water) of 200°C or lower.

An eighth aspect of the present invention is a heat exchanger comprising the chemical heat-storage material according to any one of the first to seventh aspects.

A ninth aspect of the present invention is the heat exchanger according to the eighth aspect, wherein the heat exchanger is used for a system that stores heat by desorption of water vapor (water) with heat discharged from a vehicle and generates heat by adsorption of water vapor (water).

A tenth aspect of the present invention is the heat exchanger according to the eighth aspect, wherein the heat exchanger is used for a system that stores heat by desorption of water vapor (water) with heat discharged from a factory and generates heat by adsorption of water vapor (water).

An eleventh aspect of the present invention is the heat exchanger according to the eighth aspect, wherein the heat exchanger is used for a system that stores heat by desorption of water vapor (water) with heat discharged from a device or an apparatus and generates heat by adsorption of water vapor (water).

A twelfth aspect of the present invention is a system comprising the chemical heat-storage material according to any one of the first to seventh aspects, wherein the system stores heat by desorption of water vapor (water) with heat discharged from a vehicle and generates heat by adsorption of water vapor (water).

A thirteenth aspect of the present invention is a system comprising the chemical heat-storage material according to any one of the first to seventh aspects, wherein the system stores heat by desorption of water vapor (water) with heat discharged from a factory and generates heat by adsorption of water vapor (water).

A fourteenth aspect of the present invention is a system comprising the chemical heat-storage material according to any one of the first to seventh aspects, wherein the system stores heat by desorption of water vapor (water) with heat discharged from a device or an apparatus and generates heat by adsorption of water vapor (water).

### Effects of the Invention

The heat-storage material of the present invention can adsorb or desorb water vapor (water) at a low temperature of, for example, 200°C or lower and stores a large amount of heat. Thus, the heat-storage material can be applied to a variety of products, including a portable warming tool (pocket body warmer), a food desiccant, an exothermic material in a lunch box heater, an exothermic material in a liquor heater, an adsorber for an adsorption heat pump, an adsorber for a desiccant air conditioner, a dehumidifying desiccant for an automobile, and a dehumidifying desiccant for a housing.

The heat-storage material of the present invention can serve as an efficient heat-storage material that enables utilization of absorbed heat when needed, and is suitable for use as a heat-storage material of, for example, an in-vehicle system for waste heat recovery and recycling.

### BRIEF DESCRIPTION OF THE DRAWINGS

[FIG. 1] FIG. 1 shows the results of X-ray crystallographic analysis.
[FIG. 2] FIG. 2 is a graph showing a TGA-DSC curve before a test.
[FIG. 3] FIG. 3 is a graph showing a TGA-DSC curve after a dehydration step.
[FIG. 4] FIG. 4 is a chart showing an XRD pattern before a test.
[FIG. 5] FIG. 5 is a chart showing an XRD pattern after a dehydration step.
[FIG. 6] FIG. 6 is a chart showing an XRD pattern after an adsorption step.

### MODES FOR CARRYING OUT THE INVENTION

The present invention will next be described in detail.

The chemical heat-storage material of the present invention is characterized in that it is composed of a cyanuric acid metal salt and generates or absorbs heat by adsorption or desorption of water vapor (water).

### <Chemical Heat-Storage Material>

The term "chemical heat storage" as used herein refers to a heat storage technique utilizing a reversible chemical reaction, such as generation or absorption of heat in association with a chemical change of a substance. Thus, the term "chemical heat-storage material" refers to a material that absorbs (stores) or releases heat of chemical reaction generated upon contact between a reaction medium and the heat-storage material.

In particular, the present invention is directed to a material that absorbs (stores) heat by removal of water present in a compound or generates (releases) heat by hydration of the compound; i.e., a material that generates or absorbs heat by adsorption or desorption (release) of water vapor (water).

The mechanism of generation or absorption of heat by the chemical heat-storage material referred above will now be described with reference to calcium oxide, which is a conventional typical heat-storage material, as an example.

As shown in the following (Formula 1), heat is generated when calcium oxide is transformed into calcium hydroxide by hydration reaction of calcium oxide (adsorption of water vapor (water)). Meanwhile, as shown in the following (Formula 2), heat is absorbed when calcium hydroxide is transformed into calcium oxide by dehydration of calcium hydroxide.

(Formula 1) CaO + H₂O → Ca(OH)₂ + 1.46 MJ/kg

(Formula 2) Ca(OH)₂ + 1.46 MJ/kg → CaO + H₂O

As is clear from the aforementioned (Formula 1) and (Formula 2), a heat of 1.46 MJ/kg is generated or absorbed during the hydration reaction or the dehydration reaction. The generated or absorbed heat can be considered as being stored in the chemical heat-storage material (calcium oxide).

### <Cyanuric Acid Metal Salt>

### [Cyanuric Acid]

The cyanuric acid (1,3,5-triazine-2,4,6-triol) forming the cyanuric acid metal salt usable in the present invention may be a commercially available general-purpose product. The cyanuric acid may be in the form of isocyanuric acid, which is a tautomer of cyanuric acid.

### [Metal Species]

The metal forming the cyanuric acid metal salt usable in the present invention may be any of monovalent, divalent, and trivalent metals.

The aforementioned metal is preferably at least one selected from the group consisting of lithium, sodium, potassium, magnesium, calcium, strontium, barium, aluminum, manganese, iron, cobalt, copper, nickel, zinc, silver, and tin.

The aforementioned metal is more preferably an alkaline earth metal, still more preferably at least one selected from the group consisting of magnesium and calcium.

These metals may be used alone or in combination of two or more species.

No particular limitation is imposed on the ratio by mole of cyanuric acid to the metal. When the metal is a divalent metal, such as magnesium, calcium, strontium, barium, manganese, iron (II), cobalt, copper, nickel, zinc, silver, or tin, the ratio by mole of cyanuric acid to the metal is preferably 2 : 1.

### [Production of Cyanuric Acid Metal Salt]

No particular limitation is imposed on the production method for the cyanuric acid metal salt. The cyanuric acid metal salt can be produced in the form of crystalline powder by a method involving mixing of the aforementioned cyanuric acid with a chloride, sulfate, or nitrate of any of the aforementioned metals and a base (e.g., sodium hydroxide) in water, reaction of the resultant mixture to thereby precipitate a cyanuric acid metal salt, and subsequent filtration and drying of the metal salt. Alternatively, the cyanuric acid metal salt can be produced by a method involving mixing of the aforementioned cyanuric acid with an oxide, hydroxide, or carbonate of any of the aforementioned metals in water, reaction of the resultant mixture to thereby precipitate a cyanuric acid metal salt, and subsequent filtration and drying of the metal salt.

The resultant powder is generally in the form of, for example, granular crystals, plate-like crystals, strip crystals, rod-like crystals, or acicular crystals. The powder may be in the form of a layered product of such crystals. The compound (crystalline powder) may be a commercial product if available.

The heat storage amount can be evaluated on the basis of heat balance (the amount of absorbed or released heat) as determined by differential scanning calorimetry. The chemical heat-storage material of the present invention exhibits a heat storage amount of preferably 0.5 MJ/kg or more, more preferably 0.6 MJ/kg or more, particularly preferably 0.7 MJ/kg or more. Thus, the chemical heat-storage material of the present invention preferably exhibits such a heat balance. No particular limitation is imposed on the maximum heat balance, and a higher value is more preferred. The heat balance is generally 10 MJ/kg or less.

The temperature at which the chemical heat-storage material of the present invention can adsorb or desorb water vapor (water) is preferably 200°C or lower (e.g., -30 to 200°C), more preferably 30 to 180°C, particularly preferably 50 to 100°C. When the chemical heat-storage material exhibits a heat balance within such a temperature range, the chemical heat-storage material can absorb or generate heat within the temperature range. When the chemical heat-storage material absorbs heat within the temperature range, it can be used for, for example, exhaust heat recovery or mitigation of heat island phenomenon. When the chemical heat-storage material generates heat within the temperature range, it can be used for, for example, heating or a heater of an automotive engine. Thus, the chemical heat-storage material of the present invention exhibits a heat balance within a temperature range of preferably -30 to 200°C, particularly preferably 30 to 180°C, especially preferably 50 to 100°C, as determined by differential scanning calorimetry.

The present invention also relates to a heat exchanger comprising the aforementioned chemical heat-storage material.

The present invention also relates to the aforementioned heat exchanger used for a system that stores heat by desorption of water vapor (water) with heat discharged from, for example, a vehicle, a factory, or a device or an apparatus and generates heat by adsorption of water vapor (water).

The present invention also relates to a system comprising the aforementioned chemical heat-storage material, wherein the system stores heat by desorption of water vapor (water) with heat discharged from, for example, a vehicle, a factory, or a device or an apparatus and generates heat by adsorption of water vapor (water).

In one mode of the aforementioned system, the system includes, for example, a reactor, an evaporator, a condenser, and a flow channel switching valve. The reactor is filled with the aforementioned chemical heat-storage material and is also equipped with the heat exchanger for facilitating heating or cooling.

During storage of heat, high-temperature exhaust heat from, for example, a vehicle, a factory, or a device or an apparatus is introduced into the reactor to thereby desorb water vapor (water) from the chemical heat-storage material. The resultant water vapor (water) is liquefied in the condenser. During release of heat, water vapor (water) is transferred from the evaporator to the chemical heat-storage material (from which water vapor (water) has been desorbed) to thereby adsorb water vapor (water) thereto and to generate heat. The resultant heat can be rapidly extracted via the heat exchanger to the outside of the system for use of the heat.

The aforementioned system enables efficient use of exhaust heat from a vehicle for cooling and heating, or heating of, for example, an engine, an ATF, or an exhaust gas catalyst.

### Examples

The present invention will next be described in more detail by way of examples, but the present invention is not limited to the following examples.

In the examples, the following apparatuses and conditions were used for preparation of samples and analysis of properties.

### (1) Thermogravimetry/differential scanning calorimetry (TGA-DSC)

Apparatus: TGA/DSC 1, available from Mettler-Toledo
Measurement temperature (Examples 1 to 2): 40 to 300°C
Measurement temperature (Comparative Example 1): 40 to 500°C
Heating rate: 10°C/minute
Nitrogen flow rate: 50 mL/minute
Sample pan: aluminum pan

### (2) Powder X-ray diffraction analysis (XRD)

Apparatus: desktop X-ray diffractometer MiniFlex (registered trademark) 600, available from Rigaku Corporation
Measurement angle (2θ): 3 to 40°
Scanning rate: 15°/minute

### (3) X-ray crystallographic analysis

Measurement beamline: Aichi SR BL05S2
Wavelength: 1.24 Å
Detector: PILATUS 100K
Temperature: room temperature
Exposure time: 10 minutes
Analysis: a sample encapsulated in a capillary tube having a diameter of 0.3 mm is analyzed while being rotated.
Analysis software: EXPO 2014

### [Production Example 1] Production of Magnesium Cyanurate (CA-Mg)

4.43 g (34.3 mmol) of cyanuric acid [available from Tokyo Chemical Industry Co., Ltd.] was mixed with 3.00 g (51.4 mmol) of magnesium hydroxide [available from Junsei Chemical Co., Ltd.] and 30 g of water, and the mixture was stirred at 95°C for four hours. The reaction mixture was cooled to room temperature (about 23°C), and the precipitated solid was filtered. The resultant solid was dried under reduced pressure at 50°C for one hour, to thereby produce 8.28 g of magnesium cyanurate (hydrate).

### [Production Example 2] Production of Magnesium Cyanurate (CA-Mg)

13.31 g (103.1 mmol) of cyanuric acid [available from Tokyo Chemical Industry Co., Ltd.] was mixed with 3.01 g (51.61 mmol) of magnesium hydroxide [available from Junsei Chemical Co., Ltd.] and 30 g of water, and the mixture was stirred at 95°C for three hours. The reaction mixture was cooled to room temperature (about 23°C), and the precipitated solid was filtered. The resultant solid was dried under reduced pressure at 50°C for one hour, to thereby produce 18.74 g of magnesium cyanurate (hydrate).

### [Production Example 3] Production of Magnesium Cyanurate (CA-Mg)

3.00 g (23.24 mmol) of cyanuric acid [available from Tokyo Chemical Industry Co., Ltd.] was mixed with 0.45 g (7.716 mmol) of magnesium hydroxide [available from Junsei Chemical Co., Ltd.] and 11 g of water, and the mixture was stirred at 95°C for three hours and 20 minutes. The reaction mixture was cooled to room temperature (about 23°C), and the precipitated solid was filtered. The resultant solid was dried under reduced pressure at 50°C for one hour, to thereby produce 3.77 g of magnesium cyanurate (hydrate).

### [Production Example 4] Production of Magnesium Cyanurate (CA-Mg)

3.00 g (23.24 mmol) of cyanuric acid [available from Tokyo Chemical Industry Co., Ltd.] was mixed with 0.34 g (5.830 mmol) of magnesium hydroxide [available from Junsei Chemical Co., Ltd.] and 11 g of water, and the mixture was stirred at 95°C for three hours and 20 minutes. The reaction mixture was cooled to room temperature (about 23°C), and the precipitated solid was filtered. The resultant solid was dried under reduced pressure at 50°C for one hour, to thereby produce 3.51 g of magnesium cyanurate (hydrate).

### [Production Example 5] Production of Calcium Cyanurate (CA-Ca)

3.49 g (27.0 mmol) of cyanuric acid [available from Tokyo Chemical Industry Co., Ltd.] was mixed with 3.02 g (40.8 mmol) of calcium hydroxide [available from Junsei Chemical Co., Ltd.] and 30 g of water, and the mixture was stirred at room temperature (about 23°C) for 21 hours. The precipitated solid was filtered from the reaction mixture. The solid was dried under reduced pressure at 50°C for one hour, to thereby produce 6.37 g of calcium cyanurate (hydrate).

### [Examples 1 to 5 and Comparative Example 1]

The cyanuric acid metal salts produced in Production Examples 1 to 5 and calcium hydroxide [available from Junsei Chemical Co., Ltd.] were subjected to thermogravimetry/differential scanning calorimetry for evaluation of the amount of heat absorption (heat storage amount) caused by desorption of water and heat absorption initiation temperature (desorption temperature of water). The results are shown in Table 1.

**Table 1**

| | Compound | Heat storage amount [MJ/kg] | Desorption temperature [°C] | Cyanuric acid : magnesium ratio |
|---|---|---|---|---|
| Example 1 | CA-Mg | 0.87 | 140 | 2 : 3 |
| Example 2 | CA-Mg | 1.23 | 139 | 2 : 1 |
| Example 3 | CA-Mg | 0.82 | 125 | 3 : 1 |
| Example 4 | CA-Mg | 0.62 | 121 | 4 : 1 |
| Example 5 | CA-Ca | 0.57 | 87 | - |
| Comparative Example 1 | Ca(OH)₂ | 1.34 | 427 | - |

As shown in Table 1, the heat-storage material of the present invention was found to exhibit a large heat storage amount of 0.5 MJ/kg or more and a heat storage initiation temperature (desorption temperature of water) of 200°C or lower. In particular, magnesium cyanurate (Example 2) wherein the ratio by mole of cyanuric acid to magnesium was 2 : 1 was found to exhibit very excellent performance as a heat-storage material; i.e., a heat storage amount as large as 1.23 MJ/kg and a heat storage initiation temperature of 150°C or lower.

In contrast, calcium hydroxide, which is a publicly known heat-storage material, exhibited a large heat storage amount but a very high heat storage initiation temperature of 400°C or higher. Thus, calcium hydroxide was found to be impractical (Comparative Example 1).

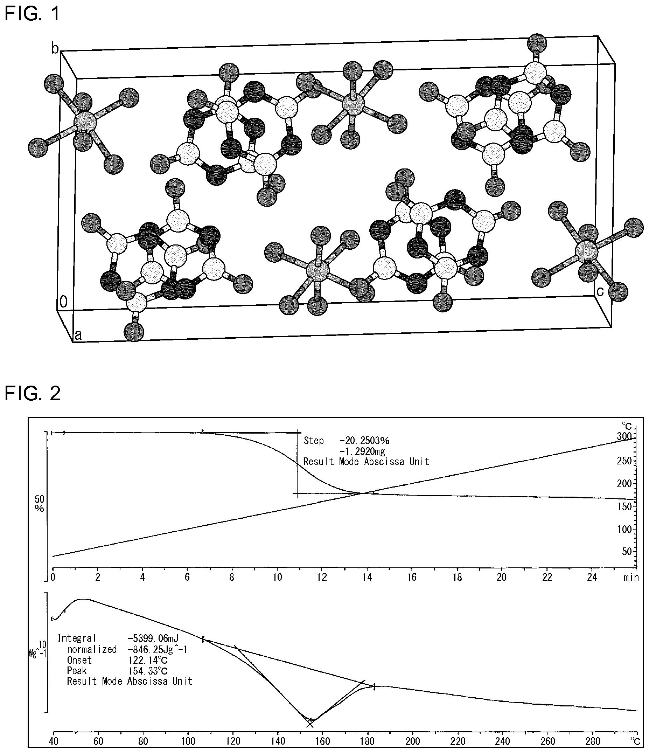

[Example 6] X-ray Crystallographic Analysis of CA-Mg The magnesium cyanurate produced in Production Example 2 (the ratio by mole of cyanuric acid to magnesium was 2 : 1) was subjected to X-ray crystallographic analysis. The results are shown in FIG. 1.

As shown in FIG. 1, the ratio of cyanuric acid to magnesium was 2 : 1 in the magnesium cyanurate produced in Production Example 2.

### [Example 7] Evaluation of Repeating Characteristics

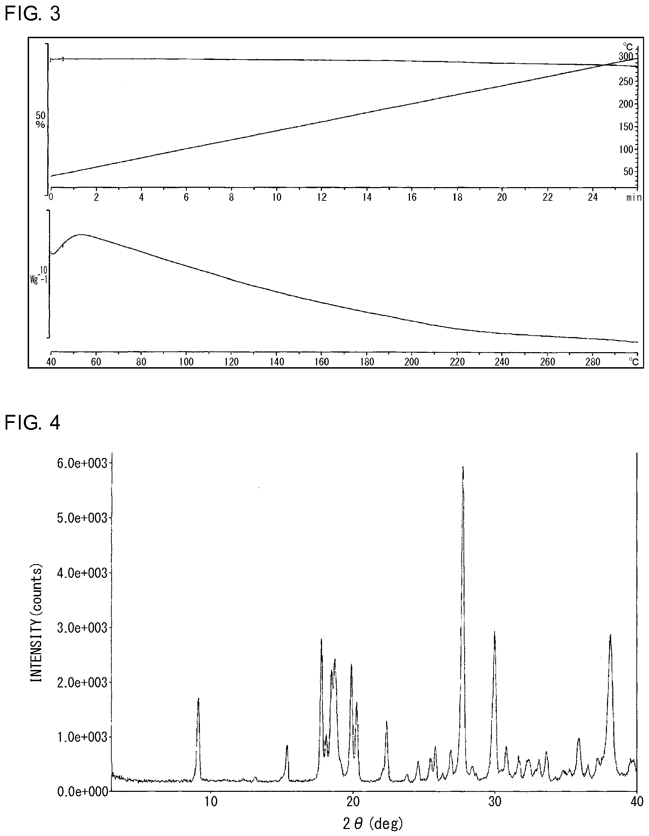

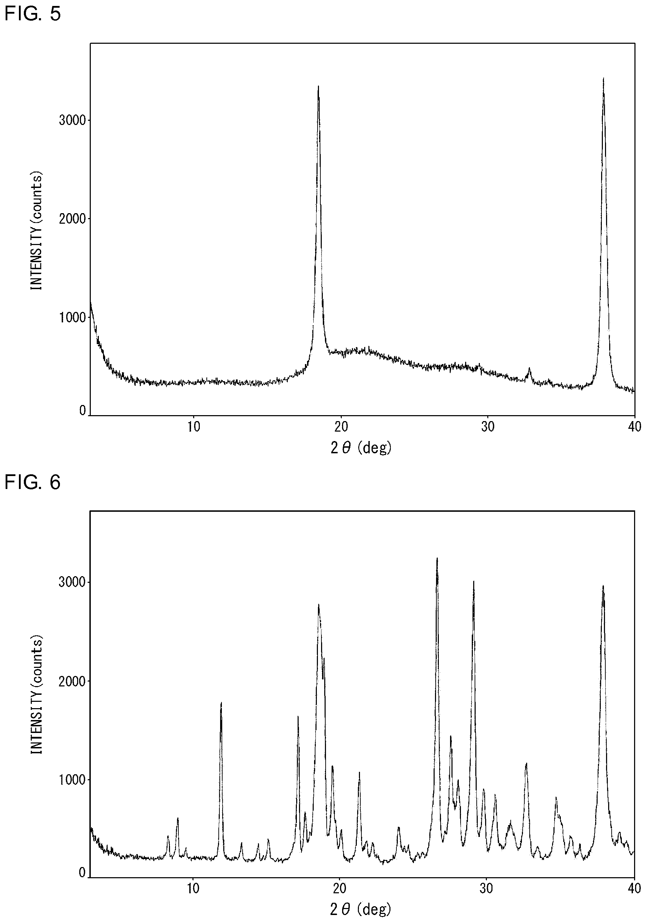

3.08 g of CA-Mg synthesized as in Production Example 1 was placed in a petri dish, and the following steps were repeated three times:
(1) dehydration step: left to stand still at atmospheric pressure at 160°C for three hours; and
(2) water vapor adsorption step: left to stand still at room temperature (about 23°C) and at 100% relative humidity for 18 hours. After completion of each step, the sample was subjected to thermogravimetry/differential scanning calorimetry and powder X-ray diffraction analysis for evaluation of the amount of heat absorption (heat storage amount) caused by desorption of water, heat absorption initiation temperature (desorption temperature of water), thermogravimetric weight loss, and XRD pattern. The results are shown in Table 2. FIG. 2 shows a TGA-DSC curve before the test; FIG. 3 shows a TGA-DSC curve after the first dehydration step; FIG. 4 shows an XRD pattern before the test; FIG. 5 shows an XRD pattern after the first dehydration step; and FIG. 6 shows an XRD pattern after the first adsorption step.

**Table 2**

| | Mass [g] | Heat storage amount [MJ/kg] | Desorption temperature [°C] | Weight loss [%] | XRD pattern |
|---|---|---|---|---|---|
| Before test | 3.08 | 0.87 | 140 | 21 | Pattern A |
| After 1^{st} dehydration step | 2.42 | No endothermic peak | | 0 | Pattern B |
| After 1^{st} adsorption step | 3.14 | 0.85 | 122 | 20 | Pattern C |
| After 2^{nd} dehydration step | 2.47 | No endothermic peak | | 0 | Pattern B |
| After 2^{nd} adsorption step | 3.11 | 0.75 | 123 | 20 | Pattern C |
| After 3^{rd} dehydration step | 2.43 | No endothermic peak | | 0 | Pattern B |
| After 3^{rd} adsorption step | 3.23 | 0.78 | 128 | 19 | Pattern C |

As shown in Table 2, the heat-storage material of the present invention underwent almost no change in heat storage performance (e.g., heat storage amount or desorption temperature) even after repetition of water desorption (dehydration)/water adsorption. Thus, the heat-storage material was found to have excellent repeating characteristics.

As described above, the heat-storage material of the present invention composed of a cyanuric acid metal salt can store a large amount of heat at a low temperature and exhibits excellent repeating characteristics. Thus, the heat-storage material is expected to achieve efficient use of unutilized waste heat.

### INDUSTRIAL APPLICABILITY

The present invention enables storage of a large amount of heat at a low temperature and achieves efficient use of unutilized waste heat.

## Claims

1. A chemical heat-storage material comprising a cyanuric acid metal salt, wherein the chemical heat-storage material generates or absorbs heat by adsorption or desorption of water vapor (water).

2. The chemical heat-storage material according to claim 1, wherein the metal species of the metal salt is at least one selected from the group consisting of lithium, sodium, potassium, magnesium, calcium, strontium, barium, aluminum, manganese, iron, cobalt, copper, nickel, zinc, silver, and tin.

3. The chemical heat-storage material according to claim 2, wherein the metal species of the metal salt is an alkaline earth metal.

4. The chemical heat-storage material according to claim 2, wherein the metal species of the metal salt is at least one selected from the group consisting of magnesium and calcium.

5. The chemical heat-storage material according to claim 4, wherein the ratio by mole of cyanuric acid to at least one selected from the group consisting of magnesium and calcium is 2 : 1.

6. The chemical heat-storage material according to any one of claims 1 to 5, wherein the amount of heat generated by adsorption of water vapor (water) or the amount of heat absorbed by desorption of water vapor (water) is 0.5 MJ/kg or more.

7. The chemical heat-storage material according to any one of claims 1 to 6, wherein the chemical heat-storage material exhibits a desorption temperature of water vapor (water) of 200°C or lower.

8. A heat exchanger comprising the chemical heat-storage material according to any one of claims 1 to 7.

9. The heat exchanger according to claim 8, wherein the heat exchanger is used for a system that stores heat by desorption of water vapor (water) with heat discharged from a vehicle and generates heat by adsorption of water vapor (water).

10. The heat exchanger according to claim 8, wherein the heat exchanger is used for a system that stores heat by desorption of water vapor (water) with heat discharged from a factory and generates heat by adsorption of water vapor (water).

11. The heat exchanger according to claim 8, wherein the heat exchanger is used for a system that stores heat by desorption of water vapor (water) with heat discharged from a device or an apparatus and generates heat by adsorption of water vapor (water).

12. A system comprising the chemical heat-storage material according to any one of claims 1 to 7, wherein the system stores heat by desorption of water vapor (water) with heat discharged from a vehicle and generates heat by adsorption of water vapor (water).

13. A system comprising the chemical heat-storage material according to any one of claims 1 to 7, wherein the system stores heat by desorption of water vapor (water) with heat discharged from a factory and generates heat by adsorption of water vapor (water).

14. A system comprising the chemical heat-storage material according to any one of claims 1 to 7, wherein the system stores heat by desorption of water vapor (water) with heat discharged from a device or an apparatus and generates heat by adsorption of water vapor (water).
